(19) Europäisches Patentamt
European Patent Office
Office européen des brevets

(11) Numéro de publication : **0 057 140**
**B1**

(12) # FASCICULE DE BREVET EUROPÉEN

(45) Date de publication du fascicule du brevet : **17.07.85**

(21) Numéro de dépôt : **82400115.0**

(22) Date de dépôt : **21.01.82**

(51) Int. Cl.⁴ : **A 61 K 37/02**, A 61 K 39/02, A 61 K 47/00, A 61 K 35/74, C 07 G 17/00, G 01 N 33/532, G 01 N 33/534, C 07 K 3/06, C 07 K 15/04

(54) **Nouveau composé polypeptidique thiolé provenant d'un fragment de la toxine tétanique, son procédé d'obtention et ses applications.**

(30) Priorité : **22.01.81 FR 8101176**

(43) Date de publication de la demande : **04.08.82 Bulletin 82/31**

(45) Mention de la délivrance du brevet : **17.07.85 Bulletin 85/29**

(84) Etats contractants désignés : **BE CH DE GB IT LI NL**

(56) Documents cités :
**DE-A- 1 617 880**
**FR-A- 2 334 954**
**FR-A- 2 366 569**
**GB-A- 1 492 445**
**US-A- 3 171 831**
**CHEMICAL ABSTRACTS, vol. 93, no. 9, 1er septembre 1980, page 111 abrégé no. 89284m COLUMBUS, Ohio (US)**
**JOURNAL OF NEUROCHEMISTRY, vol. 28, no. 3, 1977, Pergamon Press (GB) B. BIZZINI et al.: "An antigenic polypeptide fragment isolated from tetanus toxin: chemical characterization, binding to gangliosides and retrograde axonal transport in various neuron systems" pages 529-42**
**CHEMICAL ABSTRACTS, vol. 93, no. 7, 18 août 1980, page 254, abrégé no. 63204k COLUMBUS, Ohio (US) & J. Biol. Chem. 1980, 255(13), 6071-6 N.P. MORRIS et al.: "Interaction of fragments B and C of tetanus toxin with neural and thyroid membranes and with gangliosides"**

(73) Titulaire : **INSTITUT PASTEUR**
**25-28, rue du Docteur Roux**
**F-75724 Paris Cedex 15 (FR)**

(72) Inventeur : **L'inventeur a renoncé à sa désignation**

(74) Mandataire : **Phélip, Bruno et al**
**c/o Cabinet Harlé & Phélip 21, rue de la Rochefoucauld**
**F-75009 Paris (FR)**

(56) Documents cités :
**BIOCHEMISTRY, vol. 17, no. 8, 1978, American Chemical Society EASTON, Pa. (US) T.P. KING et al.: "Preparation of Protein Conjugates via Intermolecular Disulfide Bond Formation" pages 1499-1506**
**CHEMICAL ABSTRACTS, vol. 94, no. 21, 25 mai 1981, page 209, abrégé no. 169102m COLUMBUS, Ohio (US) & Brain Res. 1981, 210(1-2), 291-9 B. BIZZINI et al.: "Papain-derived fragment IIc of tetanus toxin: its binding to isolated synaptic membranes and retrograde axonal transport"**

**EP 0 057 140 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un nouveau composé polypeptide thiolé provenant d'un fragment de la toxine tétanique, son procédé d'obtention et ses applications.

En ce qui concerne la toxine tétanique elle-même ou son anatoxine on a déjà proposé de les utiliser notamment pour l'obtention de vaccins et de réactifs de dosage. Pour illustrer cet état de la technique on citera les documents ci-après :

— le certificat d'addition FR 74.16.936 publié sous le N° 2.270.891, qui concerne un procédé pour l'obtention de vaccins par traitement d'un produit toxique avec du glutaraldéhyde. Ce procédé consiste à effectuer le traitement au glutaraldéhyde. Ce procédé consiste à effectuer le traitement au glutaraldéhyde en réalisant à la fois une polymérisation d'un nombre limité de molécules du produit toxique et une détoxification dudit produit. Dans le procédé le produit toxique mis en œuvre peut être la toxine tétanique,

— la demande de brevet FR-A-2.366.569 cerne un procédé immunochimique de dosage d'haptènes, qui consiste à utiliser une particule sensibilisée par un anticorps, préparée en sensibilisant de fines particules par l'anticorps de l'haptène à doser et un conjugué haptène-support. Le support de ce conjugué haptène-support est utilisé comme réactif dans le procédé immunochimique et également pour l'immunisation d'un animal en vue de l'obtention de l'anticorps correspondant (voir page 4, lignes 20 à 32). Le toxoïde du tétanos est donc utilisé comme support de l'haptène dans l'organisme de l'animal en vue de l'obtention de l'anticorps. Cependant, rien n'indique dans cette demande de brevet qu'une fraction particulière de la toxine tétanique peut être utilisée comme agent de transport axonal de médicament,

— le brevet GB 2.013.690 concerne un antigène pour le test précoce de grossesse. Cet antigène est obtenu à partir de la sous-unité bêta de la gonadotrophine chorionique humaine, par réduction et clivage de 3, 4, 5 ou 6 ponts disulfures intrachaîne de ladite sous-unité bêta, alkylation des groupes disulfures ainsi réduits. Cet antigène peut être ensuite couplé avec une protéine ou un haptène pour augmenter sa spécificité immunologique. Le toxoïde du tétanos est cité à titre de protéine appropriée,

— le brevet GB 1.492.445 concerne une composition contenant un conjugué constitué d'un support immunogéniquement compatible et d'un dérivé de la sous-unité bêta de la gonadotrophine chorionique humaine. Le toxoïde du tétanos est utilisé comme support,

— la demande de brevet DE-OS 1.617.880 concerne un procédé pour obtenir des substances bio-actives organotropes, en particulier des médicaments. Ce procédé consiste à faire un conjugué d'une substance biologiquement active avec des substances organotropes réceptives obtenues à partir de membranes cellulaires ou d'anticorps.

A titre de substances organotropes, sont citées notamment les toxines.

Par ailleurs, on a proposé d'utiliser des protéines thiolées à titre de véhicules pour les médicaments. A cet effet on peut citer le brevet US 3.171.831 qui concerne la thiolation de protéines par réaction avec la thiolactone d'homocystéine en présence d'une amine tertiaire. Les protéines thiolées ainsi obtenues, par exemple la gélatine, peuvent être utilisées à titre de véhicules pour les médicaments. Selon l'exemple 18 de ce brevet US 3.171.831, la gélatine ainsi traitée est utilisée pour encapsuler les produits pharmaceutiques sensibles à l'environnement acide de l'estomac. Le produit pharmaceutique n'est dont pas couplé sur la protéine thiolée mais enrobé par celle-ci.

D'autre part, on a décrit dans la demande de brevet FR-A-2.334.954 un réactif pour la détermination immunologique enzymatique. Ce réactif est constitué d'un antigène et d'une enzyme couplés par l'intermédiaire d'un ester de l'acide maléimidobenzoïque et du n-hydroxy-succinimide.

On sait que la toxine tétanique est transportée de façon rétrograde vers le système nerveux central et le système nerveux périphérique. A cet effet, on peut se référer à l'article de BIZZINI et al. intitulé : « An antigenic polypeptide fragment isolated from tetanus toxin : chemical characterization, binding to gangliosides and retrograde axonal transport in various neuron systems », paru dans le « journal of Neurochemistry »; 1977, vol. 28, pp. 529-542, ainsi qu'à toutes les références bibliographiques citées dans cet article.

Différentes études ont montré que la toxine tétanique pouvait être dégradée ou scindée en plusieurs fractions ou sous-unités. Par exemple, COHEN et al. [The Journal of Immunology vol. 104, n° 6 Juin 1970] ont montré que la congélation-décongélation de filtrat brut de culture de *Clostridium tetani* entraîne la dégradation de la molécule de la toxine tétanique ; la toxine tétanique dégradée résultante est pratiquement dépourvue de toxicité et présente un pouvoir floculant plus faible que la toxine tétanique.

BIZZINI et RAYNAUD ont également étudié les sous-unités A-I, A-II, A-III, et B-I, B-II et B-III de la toxine tétanique [C.R. Acad. Sc. Paris t. 279, 1974 série D, pp. 1809-1811 et Annales de l'Institut Pasteur Paris 126, 159-176 (1975)]. Le brevet FR 74.36.622 (publication 2.249.679) décrit un produit atoxique immunogène obtenu à partir de la toxine tétanique. Ce produit atoxique est obtenu par traitement de la toxine tétanique avec une protéinase.

MORRIS et al. dans The Journal of Biological Chemistry vol. 255 n° 13 July 10, 1980 pp. 6071-6076 décrivent l'interaction des fragments B et C de la toxine tétanique avec les membranes neuronales et thyroïdes et avec les gangliosides. Il est notamment indiqué dans cet article que le fragment C, obtenu par digestion papaïnique à 55 °C, est transporté de façon rétrograde de la même façon que la toxine

tétanique intacte alors que le fragment B ne s'accumule pas de façon semblable dans le premier segment distal mais semble être absorbé ou incorporé selon un schéma diffus (voir page 6075 colonne de gauche).

Les caractéristiques physico-chimiques des différents sous-produits de la toxine tétanique sont données dans la page 230 de l'article intitulé « Tetanus Toxin » de BIZZINI dans Microbiological Reviews June 1979 p. 224-240, en particulier celles du fragment C et du fragment IIC, qui est obtenu par digestion papaïnique à une température où il n'y a pas formation du fragment C. BIZZINI et al. ont également isolé, à partir de la toxine brute congelée, un fragment polypeptidique de la toxine, qui est identique, du point de vue immunologique, aux fragments A-II et B-II cités ci-dessus, mais en diffère par sa taille et sa toxicité (voir à cet effet Journal of Neurochemistry, 1977, vol. 28, pp. 529-542). Ce fragment, dénommé B-$II_b$, est capable de se fixer sur les gangliosides et sur les membranes synaptiques avec une affinité qui est même supérieure à celle de la toxine tétanique.

Dans la demande de brevet FR-A-2.470.773 on a décrit un nouveau composé polypeptidique thiolé provenant de la toxine tétanique, son procédé d'obtention et ses applications. Ce composé polypeptidique thiolé est constitué par le fragment $BII_b$ de la toxine tétanique sur lequel est fixé au moins un groupe -SH ; ce composé présente sensiblement les mêmes propriétés de transport rétrograde axonal et de fixation sur les récepteurs de la toxine tétanique que le fragment $BII_b$ lui-même. Ce composé polypeptidique thiolé convient comme agent de transport neuropharmacologique pour véhiculer vers le système nerveux central des agents pharmacologiques ou chimiothérapeutiques. Ce composé polypeptidique convient aussi comme marqueur de cellules neuronales. Les propriétés de ce composé polypeptidique thiolé sont également décrites dans Brain Research 193 (1980) 221-227.

On a maintenant trouvé un nouveau composé polypeptidique thiolé provenant d'un autre fragment de la toxine tétanique, qui peut être également utilisé comme agent de transport neuropharmacologique pour véhiculer vers le système nerveux central des agents pharmacologiques ou chimiothérapeutiques.

La présente invention concerne donc un nouveau composé polypeptidique thiolé, son obtention et ses applications.

Le composé polypeptidique thiolé selon l'invention est constitué par le fragment $II_c$ de la toxine tétanique sur lequel est fixé au moins un groupe -SH et présente sensiblement les mêmes propriétés de transport rétrograde axonal et de fixation sur les récepteurs de la toxine tétanique que le fragment $II_c$ lui-même.

Le fragment $BII_b$ de la toxine tétanique est obtenu à partir d'un filtrat congelé de culture de *Clostridium tetani* (souche Harvard n° 6037 de la Collection Nationale de Culture de Microorganismes de l'Institut Pasteur) par ultrafiltration pour éliminer les substances ayant un poids moléculaire inférieur à 10 000, fractionnement à l'aide de sulfate d'ammonium et filtration sur gel.

Un certain nombre de fragments semblables au fragment $BII_b$ peuvent être obtenus par digestion à l'aide de papaïne, de la toxine tétanique entière ou de sa chaîne lourde (fragment bêta). A cet effet on peut se référer aux articles ci-après :

— Bizzini, B. et Raynaud, M., Etude immunologique et biologique de sous-unités de la toxine tétanique. C. R. Acad. Sci. (Paris), 279, Série D (1974) 1809-1812.

— Bizzini, B., Symposium International sur les venins et les toxines, 4-6 mars 1977, Bombay (Inde) ; résumé publié dans Toxicon (1978), volume 15, p. 141.

— Bizzini, B., Tetanus toxin. Microbiol., Rev., 43 (1979) 224-240.

Le fragment $II_c$ est obtenu par digestion de la toxine tétanique purifiée à l'aide de papaïne insolubilisée sur un gel, le « Sepharose®4B » activé avec du bromure de cyanogène selon le mode opératoire décrit dans le Compte Rendu de l'Académie des Sciences cité précédemment [t. 279 décembre 1974 ; série D-1809], si ce n'est que la digestion a été effectuée pendant 4 heures seulement.

On indiquera ci-après le mode opératoire pour obtenir le fragment $II_c$ utilisé comme composé de départ pour l'obtention du composé polypeptidique selon l'invention. On a utilisé de la toxine tétanique purifiée provenant d'un filtrat de culture de *Clostridium tetani* de 5 jours obtenu selon le procédé décrit par :

— Bizzini, B., Turpin, A. et Raynaud, M. Production et purification de la toxine tétanique, Ann. Inst. Pasteur, Paris 116 (1969) 686-712.

La toxine tétanique, purifiée ainsi obtenue titrait 3150 $L_f$ (Limes floculationis ou Unité de floculation) par mg de N et $1,2.10^8$ DMM (Dose minima mortelle) par mg de N.

120 mg de la toxine tétanique purifiée ont été traités, pendant 4 heures à 37 °C dans un bain d'eau, avec 200 unités de papaïne insolubilisée, avec agitation occasionnelle.

A la fin de la période de digestion, la papaïne a été immédiatement éliminée par centrifugation pendant 5 minutes à 10 000 g dans une centrifugeuse réfrigérée à 4 °C.

Le surnageant a été filtré sur une colonne de « Sephadex®G-100 » (2,5 × 80 cm) équilibrée avec du tampon 0,1 M Tris, HCl, pH8, contenant 1 M de NaCl. L'effluent a été continuellement contrôlé à 280 nm et recueilli par fraction de 2 ml. Les fractions correspondant au pic, ayant un poids moléculaire voisin de 45000, ont été combinées. Le volume des fractions combinées a été réduit jusqu'à 2 ml avant d'être filtré sur une colonne (2,5 × 80 cm) de gel de polyacrylamide-agarose connu sous la dénomination commerciale « Ultrogel®AcA 54 » en utilisant le même tampon. Les fractions représentant le pic principal ont été combinées et le volume résultant a été réduit jusqu'à 3 ml par dialyse contre une solution à 50 % de polyéthylèneglycol 20.000 (Sigma).

La toxine non digérée ou le fragment $I_{bc}$ qui peuvent contaminer le fragment $II_c$ ont été adsorbés sur une colonne d'affinité de gel « Sepharose » activé au bromure de cyanogène sur lequel a été couplé au préalable, de manière covalente, la fraction $I_g$G d'un sérum anti-$I_{bc}$. La fraction non adsorbée contenue dans le filtrat constitue le fragment $II_c$. Ce fragment n'est pas toxique chez la souris à la dose de 1 mg en une fois.

La colonne d'affinité a été préparée selon le mode opératoire ci-après. La fraction $I_g$G a été isolée par précipitation de l'antisérum anti-fragment $I_{bc}$. La fraction $I_g$G anti-fragment titrait 1500 $L_f$ par ml. La fraction $I_g$G, a été couplée à du « Sepharose®4B » activé au bromure de cyanogène selon le mode opératoire décrit par Wilchek et al. « A general method for the specific isolation of peptides containing modified residues, using insoluble antibody columns. Biochemistry 10 (1971) 2828-2834 ».

Le fragment $II_c$ présente les propriétés physico-chimiques ci-après :
— poids moléculaire d'environ 46 000
— il interagit avec les gangliosides
— il est dépourvu de toxicité
— il interagit avec les membranes synaptiques isolées de moelle épinière de rats.

Les propriétés immunologiques du fragment $II_c$ sont les suivantes :

le fragment $II_c$ donne une réaction croisée avec la toxine tétanique et sa chaîne lourde (fragment β). Le fragment $II_c$ donne une réaction d'identité avec le fragment B-$II_b$ ; le fragment $II_c$ donne une réaction de non identité avec le fragment $I_{bc}$ et la chaîne légère (fragment α).

Les caractéristiques principales du fragment $II_c$ sont résumées dans les articles de B. Bizzini (Microbiological Reviews, Juin 1979, p. 224-240 et Toxicon (1978), vol. 15, p. 141) dans lesquels il est indiqué que les fragments semblables au fragment $BII_b$ peuvent être obtenus par digestion à l'aide de papaïne et qu'il est probable que ces fragments contiennent la partie de la chaîne lourde que est impliquée dans la liaison de la molécule de toxine à ses récepteurs dans la cellule nerveuse. D'autre part, il est indiqué que les fragments de toxine tétanique qui possèdent encore la capacité de liaison avec les gangliosides devraient être transportés de façon rétrograde axonale comme la toxine entière.

On a maintenant trouvé que le fragment $II_c$ pouvait se fixer sur les membranes synaptiques et qu'il peut être transporté de façon rétrograde axonale, vers le système nerveux central. Le fragment $II_c$ thiolé, objet de la présente invention, est également transporté de façon rétrograde axonale vers le système nerveux central et il se fixe sur les membranes synaptiques. Etant donné que dans ce domaine une modification chimique quelconque peut inhiber les propriétés pharmacologiques d'un fragment il n'était pas du tout évident, même en présence des enseignements relatifs au composé $BII_b$, que le composé polypeptidique thiolé selon l'invention pouvait également être utilisé comme agent de transport rétrograde axonal d'agents pharmacologiques ou chimiothérapeutiques ou comme réactif de diagnostic, seul ou associé à une autre substance permettant la mise en évidence, par exemple, d'un antigène spécifique dans le système nerveux central.

La présente invention concerne donc un nouveau composé polypeptidique thiolé constitué par le fragment $II_c$ portant au moins un groupe -SH qui convient notamment à titre d'agent de transport neuropharmacologique et d'agent de marquage spécifique des cellules neuronales.

De façon analogue au procédé pour l'obtention du composé polypeptidique thiolé selon le brevet FR-A-2.470.773 le ou les groupes -SH sont fixés, directement ou indirectement, sur le fragment $II_c$. En général, compte tenu du procédé pour son obtention, qui implique une thiolation, la fixation des groupes -SH se fera par l'intermédiaire du résidu de l'agent de thiolation. Celui-ci est par ailleurs fixé sur le fragment $II_c$ par l'intermédiaire des groupes $-NH_2$ dont celui-ci est porteur.

Le composé polypeptidique thiolé selon la présente invention est obtenu par thiolation du fragment $II_c$ obtenu par le procédé décrit ci-dessus.

La thiolation du fragment $II_c$ peut être effectuée par des moyens classiques permettant l'introduction de groupes -SH sur une molécule comportant des groupes amino, mais, pour les besoins de l'invention, les moyens en cause ne doivent pas dénaturer les propriétés de transport axonal et de fixation sur les récepteurs spécifiques de la toxine tétanique dans le système nerveux central du fragment $II_c$.

A titre d'exemple, on indiquera que la thiolation du fragment $II_c$ peut être réalisée avec les agents de thiolation ci-après :

— 4-méthyl-mercaptobutyrimidate : 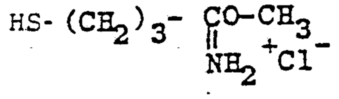

[Biochemistry vol. 17 n° 8, 1978]

— 2-iminothiolane [Schramm H.J. et Dölffer T. (1977) Z. Physiol. Chem. 358, 137-139]

— N-acéthylhomocystéine thiolactone (AHT) [voir J. Am. Chem. Soc. 1960, 82, 565-571]

(Voir dessin page 5)

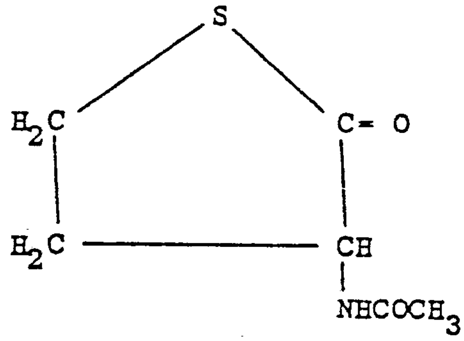

— l'anhydride S-acétyl-mercaptosuccinimique (AMS) [J. Am. Chem. Soc. 1959, 81 3802-3803]

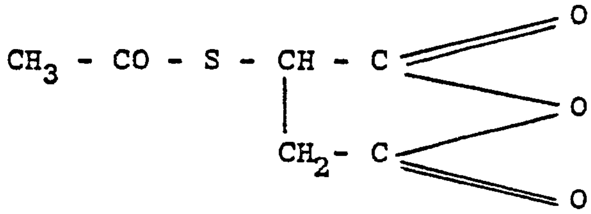

Par contre, on indiquera que les procédés connus de thiolation consistant en une étape de dithiopyridylation et en une étape de réduction ne conviennent pas aux fins de l'invention. En effet, les propriétés de transport axonal et les propriétés de fixation du fragment $II_c$ ainsi thiolé sont modifiées au cours de l'étape de réduction.

Par exemple, la thiolation effectuée par réaction avec le N-succinimidyl-3-(2-pyridyldithio)-propionate et par réduction du composé dithiopyridylé obtenu, par exemple, selon le mode opératoire décrit par CARLSSON et al [Bioch. J. (1978) 173 723-724] n'est pas appropriée aux fins de l'invention.

Pour plus de précision, on indiquera que le composé polypeptidique thiolé selon l'invention comporte un ou plusieurs groupes -Z-SH, Z étant le résidu de l'agent de thiolation.

Ainsi, si l'on met en œuvre l'un des agents de thiolation ci-dessus, —ZSH représente alors :

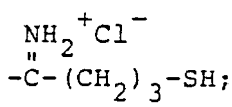

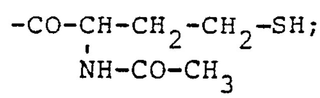

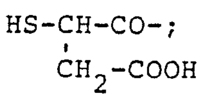

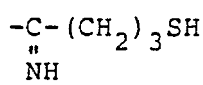

La thiolation du fragment $II_c$ est réalisée sur les groupes $—NH_2$ de celui-ci.

On a trouvé que le composé polypeptidique thiolé selon l'invention convient comme agent de transport neuropharmacologique pour véhiculer vers le système nerveux central des agents pharmacologiques ou chimiothérapeutiques.

Pour permettre le transport d'un médicament vers le système nerveux central par l'intermédiaire de l'agent selon l'invention, il est nécessaire de fixer ou de coupler ce médicament sur le composé polypeptidique thiolé, utilisé comme agent de transport, sans évidemment modifier la propriété pharmacologique du médicament ni la propriété de fixation du fragment $II_c$ sur les récepteurs spécifiques de la toxine tétanique dans le système nerveux central. Par « médicaments » on désigne, selon l'invention, toutes substances ayant des propriétés pharmacologiques, telles que les agents pharmacologiques, les agents chimiothérapeutiques, etc... Les médicaments qui peuvent être fixés selon l'invention sur le composé polypeptidique utilisé comme agent de transport neuropharmacologique doivent poséder des groupes $NH_2$.

A titre d'exemples de médicaments qui peuvent être véhiculés vers le système nerveux central au moyen du composé polypeptidique thiolé selon l'invention on citera : la phosphatase alcaline, le fragment A de la toxine cholérique, le fragment A de la toxine diphtérique, les dipyrido-indoles selon le brevet FR-A-2.387.229 et, de façon générale, tout médicament porteur de groupes $NH_2$.

Il est connu que la toxine cholérique se fixe sur les gangliosides $GM_1$ de la paroi intestinale et que le fragment A est responsable de l'augmentation du taux d'AMP cyclique [acide adénosine-monophosphorique cyclique]. Par contre, dans le tétanos, on constate une diminution du taux d'AMP cyclique dans le

système nerveux central. Le conjugué selon l'invention, constitué du composé polypeptidique thiolé couplé avec le fragment A, peut être utilisé pour lutter contre le tétanos.

Les dipyrido-indoles selon le brevet FR-A-2.387.229 sont des chimiothérapeutiques utiles dans le traitement des cancers. Dans ce domaine, on sait que les métastases sont dues au fait que les cellules cancéreuses viennent se nicher dans le système nerveux central, d'où elles migrent vers d'autres régions du corps, où elles développent des tumeurs. Le développement des métastases pourrait être évité ou réduit dès lors que les moyens pour détruire ces cellules dans le système nerveux central parviendront jusqu'au système nerveux central.

De la même manière, l'invention peut être appliquée au traitement des tumeurs cérébrales.

La présente invention concerne donc également les moyens de couplage du composé polypeptidique thiolé selon l'invention avec des médicaments.

Les moyens de couplage du composé selon l'invention et du médicament à transporter mettent en œuvre au moins un pont disulfure ou au moins une liaison irréversible soufrée.

La présente invention concerne donc également les conjugués fragment $II_c$/médicament comportant au moins un pont disulfure ou au moins une liaison irréversible soufrée.

Il est connu de préparer des conjugués protéiniques par formation de liaison disulfure intermoléculaire. La formation d'une telle liaison disulfure intermoléculaire est réalisée par exemple par réaction d'une protéine ayant des groupes thiol avec une protéine ayant des groupes dithiopyridyle.

Par exemple selon le procédé décrit par TE PIAO KING et al. (Biochemistry vol. 17 N° 8, 1978), on peut coupler deux protéines différentes en fixant d'abord sur une des protéines des groupes thiol et sur l'autre des groupes 4-dithiopyridyle et en faisant réagir les deux protéines modifiées résultantes dans des conditions appropriées permettant la formation d'un pont disulfure et l'élimination de 4-thiopyridone. Les groupes thiol peuvent être fixés sur l'une des protéines à l'aide de 4-méthylmercapto-butyrimidate et les groupes 4-dithiopyridyle sur l'autre protéine par l'intermédiaire de, par exemple, le 3-méthyl-(4'-dithiopyridyl) propionimidate. Ce procédé de couplage permet l'obtention d'un conjugué protéine-protéine dans lequel la fraction entre les deux protéines est symétrique par rapport au pont disulfure.

Selon CARLSSON et al. (Bioch. J. 1978, 173, 723-724) on peut introduire le groupe thiol sur l'une des protéines par réaction de ladite protéine avec le N-succinimidyl-3-(2-pyridyl-dithio) propionate et réduction ultérieure ; selon ce procédé on utilise le même réactif, à savoir le N-succinimidyl-3-(2-pyridyl-dithio) propionate, pour introduire des groupes thio et dithiopyridyle sur les protéines. Les conjugués résultants possèdent également une fraction de liaison symétrique par rapport au pont disulfure.

On a également utilisé le 4-méthyl-mercapto-butyrimidate pour la formation de dimères et d'oligomères supérieurs de protéines du ribosome 30 S d'*Escherichia coli* (Biochemistry, 12, 3266-3273, 1973).

Les conjugués ainsi obtenus ont de nombreuses applications, par exemple comme réactifs de dosages immunologiques.

Le procédé de couplage, selon l'invention du composé polypeptidique thiolé utilisé comme agent de transfert neuropharmacologique avec un médicament par l'intermédiaire de ponts disulfure consiste :

1. à introduire sur le médicament à fixer des groupes dithiopyridyle ;

2. à faire réagir le médicament porteur de groupes dithiopyridyle avec le composé polypeptidique thiolé selon l'invention.

Le schéma réactionnel de ce procédé de couplage peut être représenté de la façon suivante lorsque l'agent de dithiopyridylation mis en œuvre dans l'étape 1 est le N-succinimidyl-3-(2-pyridyldithio) propionate :

1) 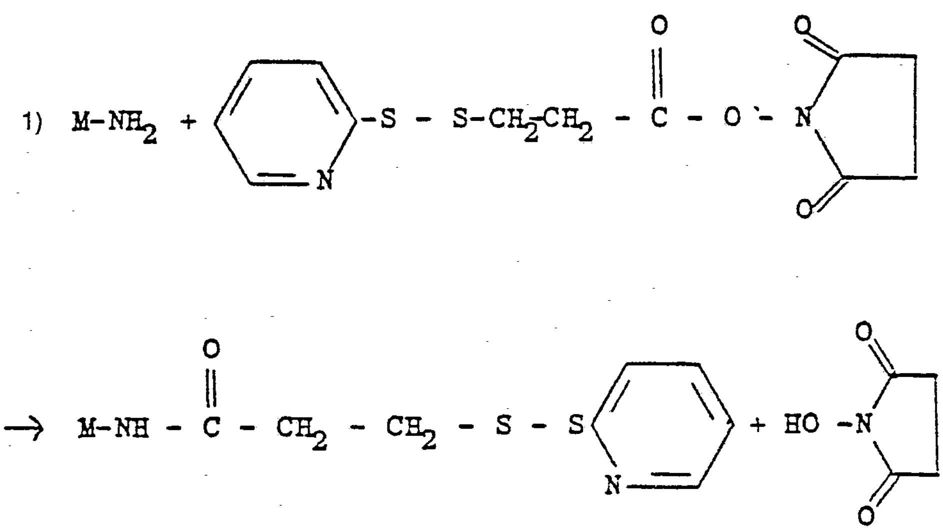

2) 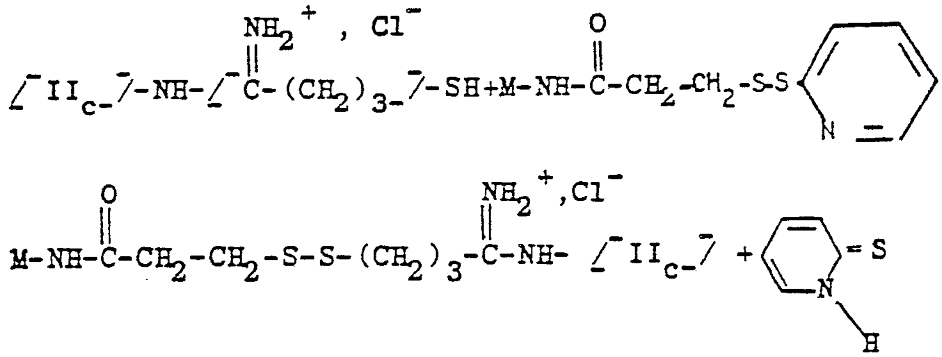

Dans ce procédé on peut utiliser un autre agent de dithiopyridylation, tel que la dithiopyridine ou tout autre agent approprié pour une telle réaction.

Un autre moyen de couplage du composé polypeptidique utilisé comme agent de transport neuropharmacologique selon l'invention consiste à créer une liaison irréversible entre ledit agent et le médicament à véhiculer. Ce procédé peut être représenté par le schéma réactionnel ci-après :

1) 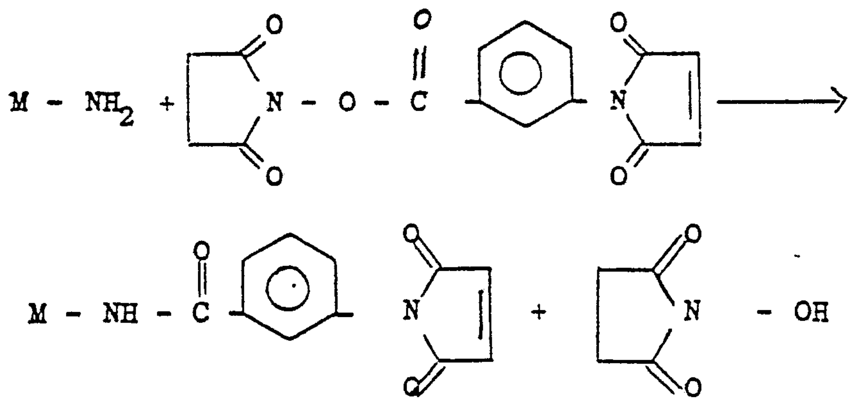

2) 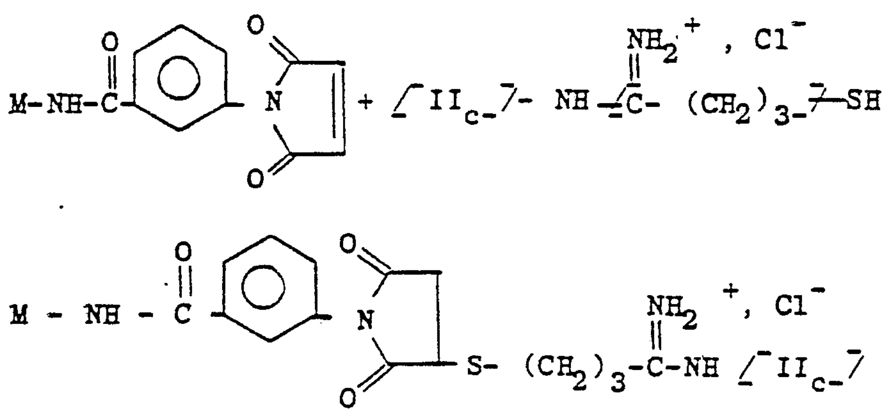

Il consiste :

1) à faire réagir le médicament à fixer avec l'ester de métamaléimidobenzoyl-N-hydroxy-succinimide,

2) à faire réagir le composé résultant avec le composé polypeptidique selon l'invention.

Les schémas réactionnels ci-dessus et ceux qui seront donnés ci-après sont simplifiés et ne tiennent pas compte du nombre de groupes SH qui peuvent être fixés sur le fragment $II_c$.

On a déjà proposé d'utiliser l'ester de métamaléimidobenzoyl-N-hydroxysuccinimide pour former des conjugués enzyme-anticorps (FEBS Letters, vol. 95, n° 2, Nov. 1978). Toutefois, les enseignements de l'art antérieur ne permettaient pas de prévoir que l'utilisation de l'ester de métamaléimidobenzoyl-N-hydroxysuccinimide pour le couplage de l'agent de transport neuropharmacologique selon l'invention avec des médicaments ne modifierait ou n'inhiberait pas les propriétés pharmacologiques dudit médicament et la propriété de fixation du fragment $II_c$ sur les récepteurs spécifiques de la toxine tétanique dans le système nerveux central.

On remarquera que le couplage selon l'invention du médicament avec le composé polypeptidique

thiolé utilisé comme agent de transport neuropharmacologique est réalisé selon des techniques connues classiques de couplage protéine-protéine. Toutefois, il faut noter que toutes les techniques de couplage protéine-protéine à la portée de l'homme de l'art ne conviennent pas aux fins de l'invention. En effet, seules les techniques de couplage permettant de réaliser une liaison disulfure ou une liaison irréversible soufrée sont appropriées. En particulier, on indiquera que la technique de couplage la plus classique, qui met en œuvre le glutaraldéhyde, ne convient pas aux fins de l'invention car le fragment $II_c$ traité par le glutaraldéhyde perd ses propriétés de transport axonal et de fixation sur les récepteurs de la toxine tétanique dans le système nerveux central. Ainsi, le fragment $II_c$, sur lequel on aurait fixé des groupes carbonyle, avec le glutaraldéhyde par exemple, ne convient pas aux fins de l'invention.

Une autre application du composé polypeptidique thiolé selon l'invention est le marquage des cellules neuronales. Ainsi, le composé de l'invention peut être utilisé comme marqueur spécifique des cellules neuronales. Il peut aussi convenir pour la préparation de réactifs immunologiques. On peut préparer par exemple un réactif enzymatique à partir du composé polypeptidique thiolé selon l'invention et de la phosphatase alcaline. On a constaté que le conjugué résultant possédait à la fois le pouvoir de fixation du fragment $II_c$ et l'activité enzymatique de la phosphatase. Le composé selon l'invention convient aussi comme traceur rétrograde transsynaptique. Le composé polypeptidique selon l'invention est également approprié pour préparer un réactif de diagnostic par couplage avec une molécule marqueur, par exemple un anticorps marqué radioactivement.

L'invention va être maintenant décrite plus en détail par les exemples illustratifs de préparation du composé polypeptidique thiolé de l'invention et de couplage de celui-ci, avec des médicaments. Dans tous ces exemples on a utilisé le fragment $II_c$ tel que défini ci-dessus.

## Exemple 1

Thiolation du fragment $II_c$ à l'aide de 2-iminothiolane.

La thiolation du fragment $II_c$ a été réalisée selon le mode opératoire décrit par Schramm et al. [Z. Physiol. Chem. 1977, *358*, 137-139].

Le fragment $II_c$ (1,5 mg) en solution dans du glycérol à 50 % (0,2 ml) a été thiolé par l'iminothiolane (2,5 mg) en solution dans 750 µl de tampon triéthanolamine HCl 0,2 M, pH 8,5-9,0. Le mélange réactionnel a été maintenu à la température ambiante pendant deux heures. L'excès de réactif a été éliminé par filtration sur une colonne « Sephadex® G-25 ». Le produit polypeptidique thiolé ainsi obtenu contenait 2,7 groupes —SH.

## Exemple 2

Thiolation du fragment $II_c$ à l'aide de N-acétylhomocystéine thiolactone.

On a réalisé la thiolation du fragment $II_c$ selon le mode opératoire décrit par SINGER et al. [J. Am. Chem. Soc. 1960, *82*, 567-571].

A 4 mg du fragment $II_c$ en solution dans 0,2 ml d'eau on a ajouté le tampon $K_2CO_3/NaHCO_3$ pH 10,7 (0,2 ml). On a fait passer un courant d'azote pour chasser l'air. On a ajouté 0,2 ml d'une solution de N-acétylhomocystéine-thiolactone à 80 mg/l. On a maintenu le mélange réactionnel pendant 2 heures à 4 °C sous azote. La réaction a été ensuite arrêtée par filtration sur Sephadex® G-25. Le produit obtenu contenait 4 groupes —SH.

## Exemple 3

Préparation d'un conjugué composé polypeptidique thiolé/fragment A de la toxine diphtérique.

On a utilisé 5 mg du fragment A de la toxine diphtérique dans 1,5 ml de tampon phosphate 0,1 M contenant NaCl 0,5 M (pH 7,5). On a ajouté 312 µg de N-succinimidyl-3-(2-pyridyl-dithio)propionate (Pharmacia) dissous dans 25 µl d'éthanol à 99,5 % (Merck). Après réaction pendant 30 minutes dans un bain-marie à 23 °C, la toxine dithiopyridylée est séparée de l'excès de réactif par filtration sur du Sephadex® G-25 équilibré avec le même tampon.

Par ce procédé, on a introduit 1,7 groupement 2-pyridyldisulfure par molécule de fragment A.

Le fragment $II_c$ a été thiolé par réaction avec du 4-méthyl-mercaptobutyrimidate selon la technique décrite par TE PIAO KING et al. (Biochemistry, vol. 17, n° 8, 1978). On a fait réagir 5,37 mg de $II_c$ dissous dans 1,5 ml de tampon borate 0,025 M pH 9,0 avec 3 mg de l'agent de thiolation dissous dans 100 µl de méthanol à 0 °C pendant 30 minutes. L'excès de réactif a été éliminé par filtration sur du Sephadex® G-25 équilibré en tampon phosphate 0,1 M pH 7,0 contenant 1 mM EDTA-Na2. Le fragment $II_c$ thiolé contenait 2,5 groupes SH par molécule.

Le conjugué a été obtenu par mélange de 119 nanomoles de fragment A dithiopyridylé avec 90,9 nanomoles de $II_c$ thiolé. On a suivi la réaction d'échange à 343 nm. On a filtré sur Sépharose® 6B équilibré avec du tampon Tris 0,05 M NaCl 0,5 M pH 8,0.

Le conjugué obtenu conserve le pouvoir de fixation du fragment $II_c$ et la réactivité immunologique du fragment A et du fragment $II_c$.

Les figures 1A et 1B illustrent la synthèse du conjugué $II_c$-polypeptide. Elles constituent le résultat de deux expériences d'immunodiffusion en gel.

La photo se trouvant à gauche de la figure 1A montre la réaction immunologique spécifique entre le fragment TT (toxine tétanique) et le sérum antitétanique mis dans le puits central. Il n'y a aucune réaction avec le fragment A de la toxine diphtérique. Le fragment A de la toxine diphtérique, dont la structure antigénique est différente de la toxine tétanique, ne donne aucune ligne de précipitation lorsqu'il est mis au contact du sérum antitétanique.

Sur la figure 1A, le composé [A-SS-$II_c$], c'est-à-dire le $II_c$-thiolé, ayant été couplé au fragment A de la toxine diphtérique, réagit avec le sérum antitétanique (SAT) par ses déterminants antigéniques tétaniques.

Sur la figure 1B, le composé [A-SS-$II_c$] réagit avec le sérum antidiphtérique (SAD) par les déterminants antigéniques portés par le fragment A de la toxine diphtérique.

Ainsi, les photos des figures 1A et 1B montrent qu'il y a eu synthèse du conjugué A-SS-$II_c$ et que les deux composants, à savoir le fragment A et le fragment $II_c$, ont conservé leurs propriétés antigéniques après couplage.

Exemple 4

Essais pharmacologiques.

Dans ces essais on a marqué à l'iode 125 le fragment $II_c$, le composé polypeptidique thiolé selon l'invention, ainsi que la toxine tétanique et les autres fragments utilisés à titre de comparaison tels que le fragment $BII_b$. Ce marquage à l'iode 125 a été effectué selon la méthode de GREENWOOD et al. [The preparation of $^{125}$I-labelled human growth hormone of high specific radioactivity. *Biochem.* J, 89 (1963) 114-127].

La radioactivité spécifique du fragment $II_c$ marqué avec $^{125}$I utilisé dans les expériences de transport rétrograde était de 4 µCi par µg de protéine. Le fragment $II_c$ utilisé dans l'essai de liaison aux membranes synaptiques avait une radioactivité spécifique de 1,2 µCi par µg de protéine. La radioactivité spécifique de la toxine tétanique était de 2,5 µCi par µg de protéine.

A) Liaison du fragment $II_c$ aux membranes synaptiques brutes.

Les membranes synaptiques brutes ont été préparées à partir de la moelle épinière de rats mâles Sprague Dawley, pesant 150-200 g. Selon la méthode de Young et Snyder, Strychnine binding associated with glycine receptors of the central nervous system. *Proc. Nat. Acad. Sci.* U.S.A., 70 (1973) 2832-2836. La concentration de protéines de la suspension de membranes a été ajustée à 1,0 mg par ml. La suspension a été stockée en parties aliquotes de 1 ml à − 25 °C. Les essais ont été réalisés selon la méthode de Young et Snyder citée ci-dessus. Les fragments $II_c$, $BII_b$, ou la toxine, ont été mis à réagir avec des parties aliquotes de 0,1 mg de membranes synaptiques brutes pendant 15 minutes à 21 °C dans 1 ml de tampon 0,05 M phosphate Na/K, pH 7,4, contenant 0,01 % de « Triton® X-100 ». La réaction a été terminée par centrifugation pendant 10 minutes à 48 000 x g et 4 °C. Le fluide surnageant a été décanté et le sédiment a été lavé 3 fois avec 5 ml de tampon 0,05 M phosphate Na/K, pH 7,4, contenant 0,01 % de « Triton® X-100 » et 0,1 % de sérum albumine bovine. On a alors mesuré la radioactivité du surnageant, des eaux de lavage et du sédiment. Chaque essai a été répété trois fois. Les essais ont été aussi réalisés en présence des fragments $II_c$ ou $BII_b$ non marqués ou de la toxine non marquée. Dans ce cas une quantité fixée de protéine marquée a été incubée avec des quantités croissantes de protéines non marquées.

Il a été vérifié que la liaison du fragment $II_c$ marqué avec $^{125}$I augmente linéairement avec la concentration en membranes.

Le déplacement du fragment $^{125}$I-$II_c$ fixé sur les membranes synaptiques isolées par le fragment $II_c$ non marqué ou la toxine tétanique marquée et le déplacement de la toxine tétanique non marquée à l'iode 125, par les fragments $II_c$ ou $BII_b$ non marqués ont été mesurés. Les résultats obtenus sont représentés sur les figures 2a et 2b sur lesquelles est indiquée en ordonnées la liaison spécifique du fragment $^{125}$I-$II_c$ (figure 2A) exprimée en cpm x $10^{-4}$ (coups par minute) ou de la toxine tétanique marquée avec $^{125}$I (figure 2B) et en abscisses la concentration molaire (M) en fragments $II_c$, B-$II_b$ ou en toxine tétanique.

Les déplacements semi-maximaux ont été calculés à partir des courbes des figures 2A et 2B et sont rassemblés dans le tableau I.

Les résultats du tableau I montrent que le déplacement semi-maximal du fragment $^{125}$I-$II_c$ lié à des membranes synaptiques a environ la même valeur qu'il soit obtenu par le fragment $II_c$ non marqué ou la toxine tétanique entière. Les résultats du tableau I montrent également que le fragment $II_c$ non marqué est aussi efficace dans le déplacement de la toxine tétanique marquée à l'iode $^{125}$I que la toxine non marquée dans le déplacement du fragment $^{125}$I-$II_c$.

Au contraire le fragment $BII_b$ est deux fois plus efficace que le fragment $II_c$ dans le déplacement de la toxine tétanique marquée à l'iode 125 liée aux membranes synaptiques.

La transformation de Hill du déplacement du fragment $^{125}I$-$II_c$ lié aux membranes synaptiques isolées par le fragment $II_c$ non marqué, exprimé en

$$Log_{10} \frac{\% \text{ B max}}{100\% - \% \text{ B max}} / Log_{10}$$

de la concentration du fragment $II_c$ non marqué donne une courbe linéaire (figure 3) avec une pente de 1,68 qui peut être interprétée comme indiquant une coopérativité positive selon Cornish-Bowden et al., Diagnostic uses of the Hill (Logit and Nernst) plots. J. Mol. Biol. 95 (1975) 201-212.

On a réalisé les mêmes essais que ci-dessus avec le fragment $II_c$ thiolé selon l'invention obtenu selon l'exemple 1 ci-dessus. Les résultats obtenus également consignés dans le tableau I montrent que la thiolation du fragment $II_c$ selon l'invention ne modifie pas essentiellement les propriétés de liaison du fragment $II_c$ sur les membranes synaptiques.

B) Transport rétrograde axonal.

Protocole d'essai.

Pour tous les essais, on a utilisé des rats Sprague-Dawley femelles d'un poids de 250 g. Les rats ont été maintenus à la température constante de 23 °C et nourris avec le régime habituel [Nafage, Gossau] et de l'eau.

Deux rats albinos ont reçu une injection du fragment $^{125}I$-$II_c$ et de peroxydase de raifort (HRP à 30 %).

A titre de témoins deux autres rats ont reçu une injection du fragment B-$II_b$ (1 µl correspondant à une concentration de 0,7 µg de protéine par µl).

Après mise à nu du muscle, les substances ont été injectées par un système d'injection réglé thermiquement à l'aide d'une pipette en verre (50-100 µm de diamètre extérieur) ; le temps d'injection a été d'environ 40 minutes.

Le volume total injecté était de 2,5 µl, soit 1,5µl de solution de fragment $II_c$ à 3 µg de protéine par µl et 1 µl de solution HRP.

Les rats ont été sacrifiés 24 heures après l'injection. Une perfusion intracardiaque a été effectuée sous anesthésie générale avec tout d'abord 0,5 ml de « Liquemine » (Roche) et 0,5 ml de nitrite de sodium (0,01 g/ml) puis ensuite avec 200 ml d'un expanseur du plasma (« MACRODEX® ») pendant 5 minutes, puis avec 600 ml de paraformaldéhyde à 1 % et de glutaraldéhyde à 2,5 % dans un tampon phosphate 0,1 M pendant 30 minutes, et finalement avec une solution de saccharose à 10 % dans un tampon phosphate 0,1 M pendant 20 minutes. Pour plus de détail sur ce mode opératoire, on pourra se reporter à l'article de MESULAM [J. Histochem. Cytochem 26, (1978) 106-117].

Le cerveau a été prélevé immédiatement après cette perfusion et maintenu pendant 48 heures dans une solution de saccharose à 30 % avant découpage. Des sections congelées (épaisseur 30 µm) ont été prélevées à partir de l'extrémité caudale du noyau abducteur et jusqu'à l'extrémité rostrale du noyau oculomoteur entier. Chaque section traitée avec la peroxydase de raifort a été colorée selon la méthode TMB selon MESULAM et recolorée avec du rouge neutre, alors que les autres sections ont été autoradiographiées. Ces dernières sections ont été montées et trempées dans une émulsion liquide $NTB_2$ à 45 °C dilué au 1/2 avec de l'eau distillée. Les sections ont été exposées pendant 4 semaines à 4 °C dans le noir et développées avec le révélateur « KODAK DEKTOL® » à 18 °C pendant 90 secondes, lavées, puis fixées avec du thiosulfate de sodium à 30 %, lavées pendant 2 heures, puis colorées avec du violet de crésyle et couvertes. Toutes les sections ont été examinées au microscope (grossissement 250) et la localisation des cellules marquées a été déterminée par microphotographie.

Comme le fragment $BII_b$, le fragment $II_c$ est transporté de façon rétrograde axonale car il se forme un marquage positif dans le noyau oculomoteur des deux animaux testés après injection de HRP et du fragment $II_c$, comme le montrent des photographies du complexe oculomoteur avec un marqueur radioactif prises dans la région considérée.

La principale différence entre le HRP et le fragment $II_c$ réside dans le fait que la localisation des granules HRP est limitée au péricarya et aux dendrites des neurones oculomoteurs, tandis que des grains d'argent représentant le fragment $^{125}I$-$II_c$ sont également situés dans les espaces péricellulaires.

Par rapport au fragment B $II_b$ et ses conjugués, le fragment $II_c$ semble être localisé plus strictement dans les corps cellulaires des neurones moteurs marqués que dans les espaces extracellulaires. Bien qu'une activité totale supérieure ait été injectée dans les deux cas avec le fragment $II_c$ on a noté un marquage moins intensif du noyau oculomoteur par rapport au fragment B $II_b$.

Le fait que le transport rétrograde axonal est directement lié à la capacité des fragments à se fixer sur leurs récepteurs membranaires, indique clairement que le fragment $II_c$ thiolé ayant conservé ses propriétés de fixation (tableau I) est également transporté par voie rétrograde.

On a aussi vérifié, en utilisant les tests décrits dans Journal of Neurochemistry (1977) vol. 28 p. 529-542, que les conjugués $II_c$/médicament obtenus selon l'invention à partir du composé polypeptidique

thiolé se fixaient de façon spécifique sur les gangliosides et les membranes synaptiques ; ces résultats prouvent que ces substances peuvent donc être transportées de façon rétrograde axonale.

Tableau I

Déplacements semi-maximaux par mg de protéines de membrane calculés à partir des courbes des figures 1A et 1B

| Déplacement | Déplacement semi-maximal nM/mg |
|---|---|
| $^{125}$I-$II_c$-fragment $II_c$ non marqué | 8,0 |
| $^{125}$I-$II_c$-toxine tétanique non marquée | 9,0 |
| $^{125}$I-toxine tétanique-fragment $II_c$ non marqué | 9,0 |
| $^{125}$I-toxine tétanique-fragment B-$II_b$ non marqué | 5,5 |
| $^{125}$I-$II_c$ thiolé-fragment $II_c$ non marqué | 9,5 |

**Revendications**

1. A titre de produit nouveau, le composé polypeptidique thiolé constitué par le fragment $II_c$ de la toxine tétanique, sur lequel est fixé, directement ou indirectement, au moins un groupe —SH.

2. Composé polypeptidique thiolé selon la revendication 1, caractérisé en ce qu'il est obtenu par thiolation et en ce qu'il comporte un ou plusieurs groupes Z—SH, Z étant le résidu de l'agent de thiolation.

3. Composé selon l'une des revendications 1 ou 2, caractérisé en ce que —ZSH est choisi parmi les groupes ci-après :

```
 NH2+Cl-
 ||
-C-(CH2)3-SH;              -CO-CH-CH2-CH2SH;
                               |
                               NH-CO-CH3


HS-CH-CO-;                 -C-(CH2)3SH
   |                        ||
   CH2-COOH                 NH
```

4. Procédé d'obtention d'un produit selon l'une quelconque des revendications 1 à 3, caractérisé en ce que la thiolation du fragment $II_c$ est réalisée à température comprise entre 0 °C et la température ambiante et à un pH compris entre 6,7 et 9, en présence d'un solvant organique pour permettre de conserver les propriétés du fragment $II_c$ de transport rétrograde axonal et de fixation sur les récepteurs spécifiques de la toxine tétanique dans le système nerveux central.

5. Procédé selon la revendication 4, caractérisé en ce que l'agent de thiolation est choisi parmi :
le 4-méthyl-mercaptobutyrimidate,
le 2-iminothiolane,
la N-acétylhomocystéine thiolactone,
l'anhydride S-acétyl-mercaptosuccinimique.

6. Application du composé polypeptidique thiolé selon l'une quelconque des revendications 1 à 3 à titre d'agent de transport neuropharmacologique spécifique pour véhiculer un médicament dans le système nerveux central.

7. Application du composé polypeptidique thiolé selon l'une quelconque des revendications 1 à 3 à titre de marqueur spécifique des cellules neuronales.

8. Procédé de couplage d'une substance avec le composé polypeptidique thiolé selon l'une quelconque des revendications 1 à 3, caractérisé en ce qu'il consiste :

1) à introduire sur la substance à fixer des groupes dithiopyridyle ;

2) à faire réagir la substance porteur de groupes dithiopyridyle avec le composé polypeptidique thiolé selon l'une quelconque des revendications 1 à 3.

9. Procédé selon la revendication 8, caractérisé en ce que, dans l'étape (1) on utilise un agent de dithiopyridylation, tel que le N-succinimidyl-3-(2-pyridyl-dithio) propionate ou la dithiopyridine.

10. Procédé de couplage d'une substance avec le composé polypeptidique thiolé selon l'une quelconque des revendications 1 à 3, caractérisé en ce qu'il consiste à :

1) faire réagir la substance à fixer avec l'ester de métamaléimidobenzoyl-N-hydroxy-succinimide ;

2) à faire réagir le composé résultant avec le composé polypeptidique thiolé selon l'une quelconque des revendications 1 à 3.

11. Procédé selon l'une quelconque des revendications 8 à 10, caractérisé en ce que la substance est choisie parmi les médicaments et les marqueurs.

12. Conjugué fragment $II_c$/médicament comportant au moins un pont disulfure.

13. Conjugué fragment $II_c$/médicament comportant au moins une liaison irréversible —S—.

14. Conjugué fragment $II_c$/médicament obtenu par le procédé selon l'une quelconque des revendications 8 à 10.

15. Conjugué fragment $II_c$/médicament selon l'une quelconque des revendications 12 à 14, caractérisé en ce que le médicament est une substance portant des groupes $—NH_2—$.

16. Conjugué fragment $II_c$/médicament selon l'une quelconque des revendications 11 à 14, caractérisé en ce que le médicament est le fragment A de la toxine cholérique, le fragment A de la toxine diphtérique, un dipyrido-indole.

17. Conjugué fragment $II_c$/marqueur comportant au moins un pont disulfure.

18. Conjugué fragment $II_c$/marqueur comportant au moins une liaison irréversible —S—.

19. Conjugué selon l'une des revendications 17 ou 18, caractérisé en ce que le marqueur est une substance marquée radioactivement, par exemple un anticorps.

20. Application du conjugué selon l'une quelconque des revendications 17 à 19 comme réactif de diagnostic.

21. Application selon la revendication 20, caractérisée en ce que ladite substance marquée radioactivement est un anticorps.

**Claims**

1. As new product, a thiolated polypeptidic compound consisting of the $II_c$ fragment of tetanus toxin to which is bound, directly or indirectly, at least one —SH group.

2. A thiolated polypeptidic compound according to claim 1, characterized in that it results from a thiolating reaction and comprises one or more —ZSH groups bound, wherein Z is the residue from the thiolating agent.

3. A compound according to claims 1 or 2, characterized in that —ZSH is selected from :

$$-\underset{\|}{\overset{NH_2^+Cl^-}{C}}-(CH_2)_3-SH; \qquad -CO-\underset{NH-CO-CH_3}{\underset{|}{CH}}-CH_2-CH_2-SH;$$

$$HS-\underset{CH_2-COOH}{\underset{|}{CH}}-CO-; \qquad -\underset{NH}{\underset{\|}{C}}-(CH_2)_3-SH$$

4. A process for producing a compound according to any claim 1 to 3, characterized in that the thiolating reaction of $II_c$ fragment is performed at a temperature in the range of 0 °C to ambient

temperature, at pH from 6,7 to 9, in presence of an organic solvent, so that the $II_c$ fragment properties, consisting of retrograde axonal transport activity and binding to central nervous system specific receptors of tetatus toxin, are still present.

5. A process according to claim 4, characterized in that the thiolating agent is selected from :
4-methylmercaptobutyrimidate
2-iminothiolane
N-acetylhomocysteine thiolactone
S-acetylmercaptosuccinimic acid anhydride.

6. Use of thiolated polypeptidic compound according to any claim 1 to 3, as specific neuropharmacological vehicle for transporting a medicine to the central nervous system.

7. Use of thiolated polypeptidic compound according to any claim 1 to 3 as specific labeller of neurone cells.

8. A process for coupling a substance to the thiolated polypeptidic compound according to any claim 1 to 3 characterized in that it comprises the stages :

1) of fixing dithiopyridyl groups on the substance to be coupled,

2) of reacting the dithiopyridyl-reacted substance with the thiolated polypeptidic compound according to any claim 1 to 3.

9. A process according to claim 8, characterized in that in stage 1 the agent for fixing dithiopyridyl groups is selected from N-succinimidyl-3-(2-pyridyldithio)propionate or dithiopyridine.

10. A process for coupling a substance to the thiolated polypeptidic compound according to any claim 1 to 3, characterized in that it comprises the stages :

1) of reacting the substance to be coupled with meta-maleimidobenzoyl-N-hydroxysuccinimide ester ;

2) of reacting the resulting compound with the thiolated polypeptidic compound according to any claim 1 to 3.

11. A Process according to any claim 8 to 10, characterized in that the substance is selected from medicines or labellers.

12. A $II_c$ fragment/medicine conjugate comprising at least one disulphide coupling group.

13. A $II_c$ fragment/medicine conjugate comprising at least one stable —S-bond.

14. A $II_c$ fragment/medicine conjugate resulting from the process according to any claim 8 to 10.

15. A $II_c$ fragment/medicine conjugate according to any claim 12 to 14, characterized in that said medicine is a compound having $—NH_2$ groups.

16. A $II_c$ fragment/medicine conjugate according to any claim 11 to 14, characterized in that said medicine is selected from A fragment of cholera toxin, A fragment of diphteria toxin or a dipyridoindole.

17. A $II_c$ fragment/labeller conjugate having at least one disulphide coupling group.

18. A $II_c$ fragment/labeller conjugate having at least one stable —S-bond.

19. A conjugate according to claim 17 or 18, characterized in that the labeller is a radioactive labeller, such as an antibody.

20. Use of conjugate according to any claim 17 to 19, as diagnosis reagent.

21. Use according to claim 20, characterized in that said radioactive labeller is an antibody.

**Patentansprüche**

1. Neues Produkt in Form einer Polypeptidverbindung mir Thiol-Gruppe(n), bestehend aus dem Fragment $II_c$ des Tetanus-Toxins, an das unmittelbar oder mittelbar mindestens eine SH-Gruppe gebunden ist.

2. Polypeptidverbindung mit Thiol-Gruppe(n) nach Anspruch 1, dadurch gekennzeichnet, daß sie durch eine Reaktion zur Einführung von Thiolgruppen erhalten wurde und eine oder mehrere Gruppen der Formel —Z—SH enthält, wobei Z für den Rest des Reagens zur Einführung der Thiolgruppen steht.

3. Verbindung nach einem der Ansprüche 1 oder 2, dadurch gekennzeichnet, daß die Gruppe —Z—SH ausgewählt ist aus den nachfolgenden Resten :

$$-\overset{\overset{\displaystyle NH_2^+Cl^-}{\|}}{C}-(CH_2)_3-SH; \qquad -CO-\underset{\underset{\displaystyle NH-CO-CH_3}{|}}{CH}-CH_2-CH_2SH;$$

$$HS-\underset{\underset{\displaystyle CH_2-COOH}{|}}{CH}-CO-; \qquad -\underset{\underset{\displaystyle NH}{\|}}{C}-(CH_2)_3SH$$

4. Verfahren zur Herstellung eines Produktes nach einem der Ansprüche 1 bis 3, dadurch

gekennzeichnet, daß man die Einführung der Thiolgruppen in das Fragment $II_c$ bei einer Temperatur im Bereich von 0 °C bis Raumtemperatur und einem pH-Wert im Bereich von 6,7 bis 9 durchführt in Gegenwart eines organischen Lösungsmittels, um die Erhaltung der Eigenschaften des Fragments $II_c$ eines axonalen retrograden Transports und einer Fixierung an den für das Tetanus-Toxin spezifischen Rezeptoren im zentralen Nervensystem zu ermöglichen.

5. Verfahren nach Anspruch 4, dadurch gekennzeichnet, daß das Reagens zur Einführung der Thiolgruppe ausgewählt wird aus den Verbindungen
4-Methylmercaptobutyrimidat,
2-Iminothiolan,
N-Acetylhomocystein-thiolacton und
S-Acetylmercaptobernsteinsäureanhydrid.

6. Verwendung der Polypeptidverbindungen mit Thiol-Gruppe(n) nach einem der Ansprüche 1 bis 3 als spezifisches neuropharmakologisches Transport-Agens zur Beförderung eines Arzneimittels in das zentrale Nervensystem.

7. Verwendung der Polypeptidverbindungen mit Thiol-Gruppe(n) nach einem der Ansprüche 1 bis 3 als spezifischer Marker für neuronale Zellen.

8. Verfahren zur Kupplung einer Substanz an Polypeptidverbindungen mit Thiol-Gruppe(n) nach einem der Ansprüche 1 bis 3, gekennzeichnet durch die Verfahrensschritte :
1) Einführung von Dithiopyridyl-Gruppen in die zu fixierende Substanz,
2) Reaktion der Dithiopyridyl-Gruppen tragenden Substanz mit einer Polypeptidverbindung mit Thiol-Gruppe(n) nach einem der Ansprüche 1 bis 3.

9. Verfahren nach Anspruch 8, dadurch gekennzeichnet, daß man im Verfahrensschritt (1) ein Reagens zur Einführung von Dithiopyridyl-Gruppen, wie N-Succinimidyl-3-(2-pyridyldithio-) propionat oder Dithiopyridin, verwendet.

10. Verfahren zur Kupplung einer Substanz mit einer Polypeptidverbindung mit Thiol-Gruppe(n) nach einem der Ansprüche 1 bis 3, gekennzeichnet durch die Verfahrensschritte :
1) Reaktion der zu bindenden Substanz mit dem N-Hydroxybernsteinsäureester der m-Maleinimidobenzoesäure,
2) Reaktion der resultierenden Verbindung mit der Polypeptidverbindung mit Thiol-Gruppe(n) nach einem der Ansprüche 1 bis 3.

11. Verfahren nach einem der Ansprüche 8 bis 10, dadurch gekennzeichnet, daß die Substanz aus der Gruppe der Arzneimittel und Marker ausgewählt ist.

12. Konjugat aus dem Fragment $II_c$ und einem Arzneimittel, enthaltend wenigstens eine Disulfid-Brücke.

13. Konjugat aus dem Fragment $II_c$ und einem Arzneimittel, enthaltend wenigstens eine irreversible —S-Bindung.

14. Konjugat aus dem Fragment $II_c$ und einem Arzneimittel, erhalten nach dem Verfahren gemäß einem der Ansprüche 8 bis 10.

15. Konjugat aus dem Fragment $II_c$ und einem Arzneimittel nach einem der Ansprüche 12 bis 14, dadurch gekennzeichnet, daß das Arzneimittel eine Substanz ist, die —$NH_2$-Gruppen trägt.

16. Konjugat aus dem Fragment $II_c$ und einem Arzneimittel nach einem der Ansprüche 11 bis 14, dadurch gekennzeichnet, daß das Arzneimittel das A-Fragment des Cholera-Toxins, das A-Fragment des Diphterie-Toxins oder ein Dipyridoindol ist.

17. Konjugat aus dem Fragment $II_c$ und einem Marker, enthaltend wenigstens eine Disulfid-Brücke.

18. Konjugat aus dem Fragment $II_c$ und einem Marker, enthaltend wenigstens eine irreversible—S-Bindung.

19. Konjugat nach einem der Ansprüche 17 oder 18, dadurch gekennzeichnet, daß der Marker eine radioaktiv markierte Substanz, beispielsweise ein Antikörper ist.

20. Verwendung der Konjugate nach einem der Ansprüche 17 bis 19 als Reagens für die Diagnostik.

21. Verwendung entsprechend Anspruch 20, dadurch gekennzeichnet, daß die genannte radioaktiv markierte Substanz ein Antikörper ist.

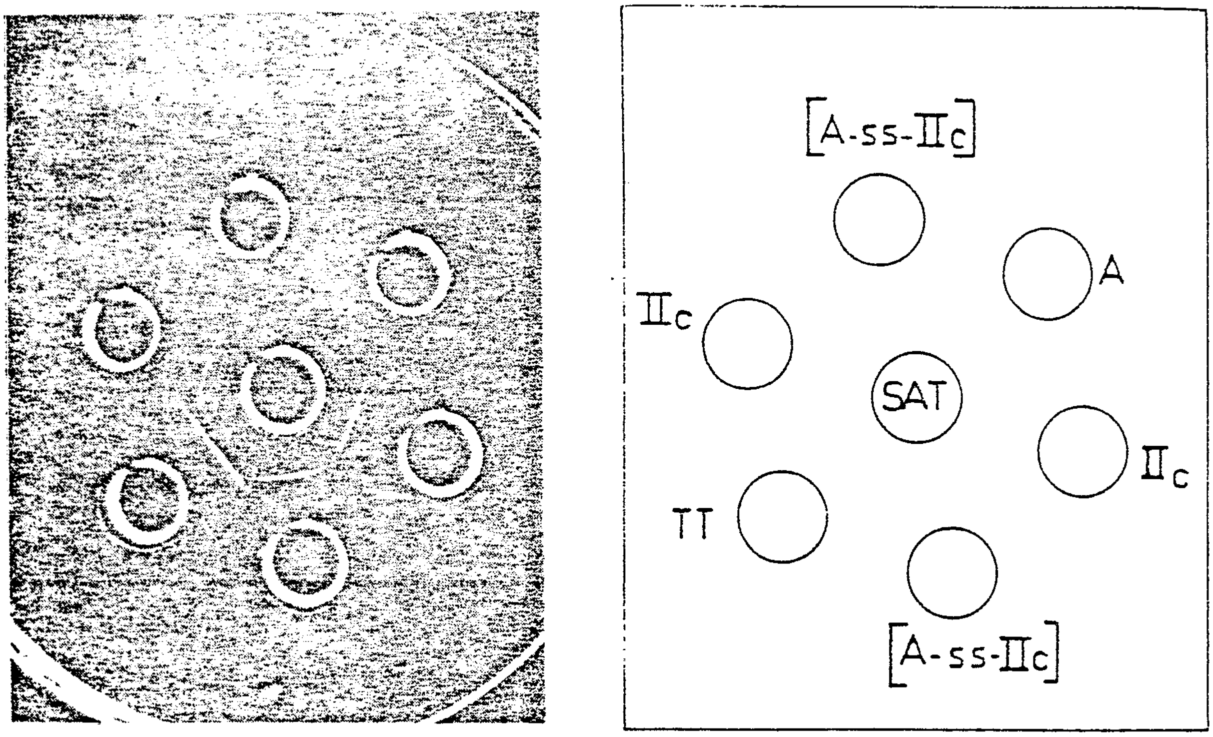


FIG.1A

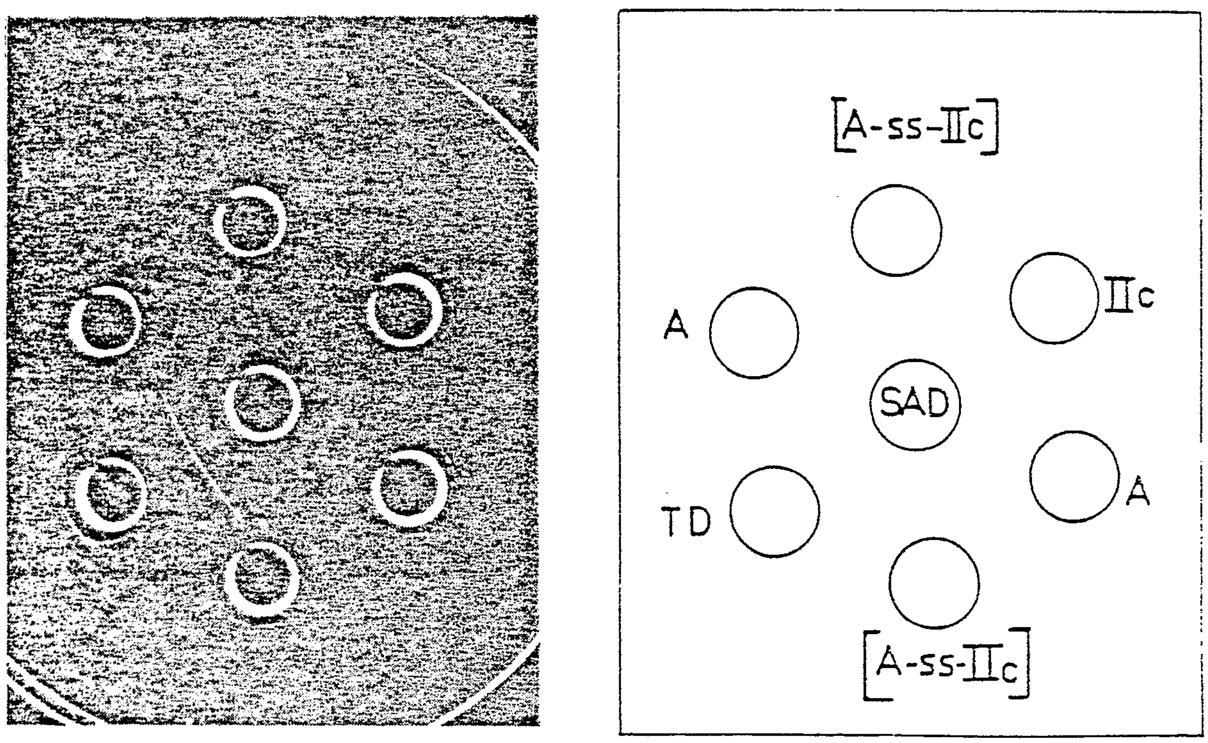


FIG.1B

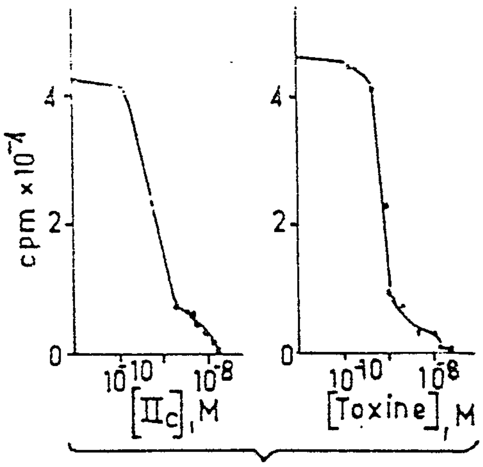


FIG. 2A

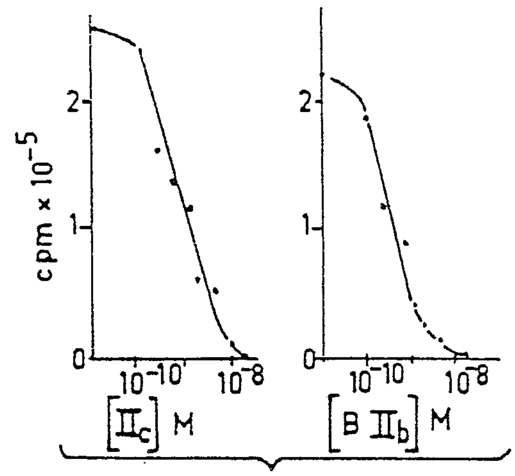


FIG. 2B

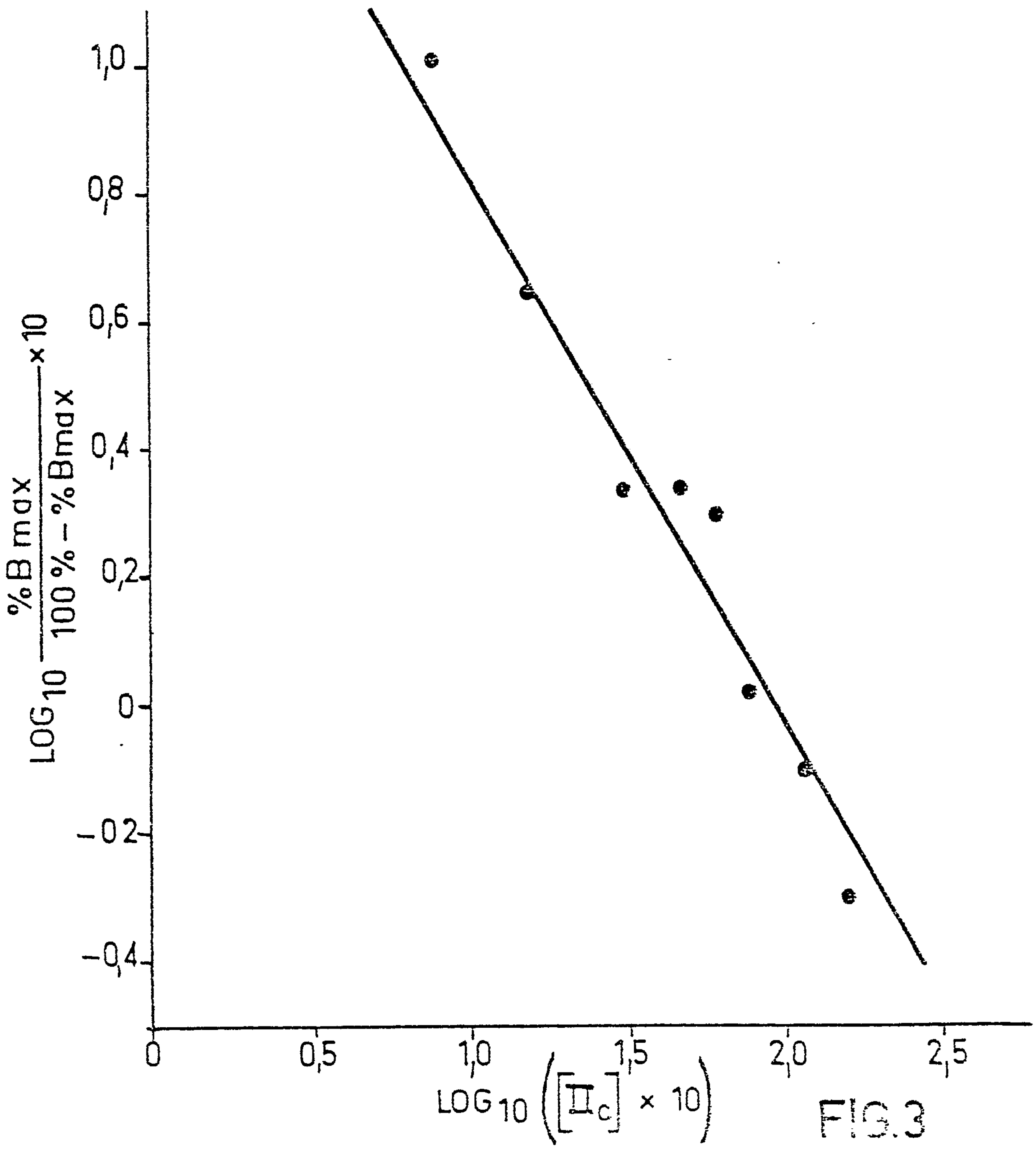


FIG. 3